# EUROPEAN PATENT SPECIFICATION

(11) **EP 4 421 083 B1**
(45) Date of publication and mention of the grant of the patent: **07.10.2026**
(21) Application number: 21962856.7
(22) Date of filing: 04.11.2021
(51) Int. Cl.: C07K 7/00, A61K 8/64, A61Q 19/00, A61Q 19/08, A61P 17/00, A61P 17/18

(54) **HEXAPEPTIDE DERIVATIVE AND COSMETIC COMPOSITION OR PHARMACEUTICAL COMPOSITION AND USE THEREOF**
HEXAPEPTIDDERIVAT UND KOSMETISCHE ZUSAMMENSETZUNG ODER PHARMAZEUTISCHE ZUSAMMENSETZUNG UND VERWENDUNG DAVON
DÉRIVÉ D'HEXAPEPTIDE ET COMPOSITION COSMÉTIQUE OU COMPOSITION PHARMACEUTIQUE ET UTILISATION ASSOCIÉE

(43) Date of publication of application: 28.08.2024
(73) Proprietor: Shenzhen Winkey Technology Co., Ltd, Shenzhen, Guangdong 518000 (CN)
(72) Inventor: DING, Wenfeng, Shenzhen, Guangdong 518100 (CN); PENG, Yan, Shenzhen, Guangdong 518100 (CN); CHEN, Xue, Shenzhen, Guangdong 518100 (CN); ZHAO, Wenhao, Shenzhen, Guangdong 518100 (CN); SUN, Xinlin, Shenzhen, Guangdong 518100 (CN); HUANG, Chunqing, Shenzhen, Guangdong 518100 (CN); GUAN, Fuyi, Shenzhen, Guangdong 518100 (CN); XIAO, Yu, Shenzhen, Guangdong 518100 (CN)
(74) Representative: Bayramoglu et al.
(86) International application number: PCT/CN2021/128560
(87) International publication number: WO 2023/077338

(56) References cited:
- WO-A2-2006/048339
- CN-A- 101 474 153
- CN-A- 102 440 943
- CN-A- 102 824 278
- KR-B1- 101 142 044
- KR-B1- 101 354 915
- US-A1- 2004 142 873
- US-A1- 2010 003 344
- US-A1- 2010 261 879
- US-A1- 2013 079 287
- US-A1- 2014 056 835

## Description

### TECHNICAL FIELD

The present invention relates to a peptide derivative, a cosmetic composition or pharmaceutical composition comprising the peptide derivative and use thereof.

### BACKGROUND

The skin is the largest and most important organ of the human body, covering the entire surface of the human body and consisting of epidermis, dermis and subcutaneous tissue. The epidermis and dermis are connected by a basement membrane, and the structural components of the basement membrane include type VII collagen, type IV collagen, laminin, integrin, endonexin, heparan sulfate glycoprotein, etc.

Fibroblasts in the dermis can promote collagen synthesis, and collagen molecules are intertwined to form fibers, forming a fine mesh of collagen fibers in the skin to increase the connection strength and stability of the skin. Collagen is an important component of the human skin. It has a significant water-locking function, which directly determines the moisture, smoothness, firmness and age of the skin.

Skin aging is affected by endogenous and exogenous factors. With advancing age and long-term exposure to the sun or UV radiation, collagen will be lost in a large amount, and at the same time, the collagen synthesized by fibroblasts will also decrease. These cause various peptide bonds of collagen that support the skin to harden and break, so that the reticular structure of the dermis of the skin loosens, and then is destroyed and collapsed. The moisture retention and strength of the skin tissue decrease, and skin problems such as dryness, wrinkles, and sagging subsequently appear.

H-Gly-Pro-Gln-Gly-Pro-Gln-NH₂ (EP1406589B1) is a collagen peptide, whose structure is found in the structures of the body's two key basement membrane collagen types IV and XVII simultaneously. It can enhance skin intercellular adhesion, increase the contents of type I collagen, type IV collagen, laminin-5, and integrin, promote the differentiation and maturation of epidermal cells, and boost skin regeneration, thereby exerting anti-aging effects.

In addition to age factors and UV radiation, runaway oxygen free radicals also play an important role in skin aging. Under harmful stimuli, when reactive oxygen species (ROS) are generated too much or suffer from metabolic disorders and exceed the clearance capacity of the endogenous antioxidant defense system, ROS will increase in the body, participate in the formation of oxidized biomacromolecules, and directly or indirectly oxidize or damage DNA, proteins and lipids, eventually leading to oxidative damage of cells. ROS attacks the cell membrane of normal cells, causing increased membrane permeability; or attacks fibroblasts, resulting in abnormal collagen metabolism, decreased collagen synthesis, and abnormal crosslinking. Elastic fibers are also denatured and curled under the attack of oxygen free radicals, thus further affecting the dermis structure and accelerating skin aging.

Caffeic acid and gallic acid have phenolic hydroxyl groups in the molecular structures and have strong reducibility. They can effectively capture oxygen free radicals and interrupt chain reactions, thereby scavenging free radicals and protecting the body from oxidative damage. They are widely used in the fields of medicines and cosmetics.

Caffeic acid (CAS No.: 331-39-5), also known as hydrolyzed chlorogenic acid or 3,4-dihydroxycinnamic acid, is a naturally occurring phenolic acids compound, commonly found in various foods such as coffee drinks, apples, and blueberries. In recent years, with in-depth research on the biological activity of caffeic acid, caffeic acid is also found to have good applications in cosmetics. Caffeic acid has good antioxidant activity and can protect the skin from UV rays. Therefore, caffeic acid can be used as an anti-aging and sunscreen ingredient in cosmetics.

Gallic acid (CAS No.: 149-91-7), whose scientific name is 3,4,5-trihydroxybenzoic acid, is widely present in plants such as *Rheum palmatum, Eucalyptus robusta Smith* and *Cornus officinalis,* and is a polyphenolic compound that exists in nature. 95% Gallic acid has been used as an antioxidant in Japan in the 1980s. At present, gallic acid and derivatives thereof are widely used as free radical scavengers in the fields of biology, medicine, food, and chemical industry.

Peptides with cosmetic activity and an N-terminal caffeoyl or galloyl-group modification are known in the art (see for example KR 101 142 044 B1, KR 101 354 915 B1, US 2013/079287 A1, US 2014/056835 A1, US 2010/261879 A1).

In order to enhance the anti-aging effect, it is theoretically considered to use H-Gly-Pro-Gln-Gly-Pro-Gln-NH₂ in combination with caffeic acid and gallic acid. H-Gly-Pro-Gln-Gly-Pro-Gln-NH₂ has good solubility in water, while caffeic acid is slightly soluble in cold water, and gallic acid is insoluble in cold water. Dissolution with heating may affect the stability of polypeptides. Moreover, caffeic acid and gallic acid have certain cytotoxicity. In fact, the feasibility of the combined formulation is yet to be studied, and the effects of combined use are yet to be verified. Therefore, there is a need to study a novel compound having highly efficient anti-photoaging and anti-oxidation effects, thereby overcoming the existing defects in the prior art.

### SUMMARY

Through experimental research, the inventors of the present invention linked Gly-Pro-Gln-Gly-Pro-Gln with caffeic acid or gallic acid via an amide bond to obtain the peptide derivative of the present invention, and unexpectedly found that compared with Gly-Pro-Gln-Gly-Pro-Gln, caffeic acid or gallic acid alone, the peptide derivative of the present invention has greatly improved anti-photoaging repair ability and antioxidant effect, and achieved unexpected technical effects. These peptide derivatives, as well as cosmetic compositions or pharmaceutical compositions comprising these peptide derivatives can be used to treat, prevent and/or repair signs of aging and/or photoaging of the skin, exert antioxidant effects by scavenging free radicals, and can be used in cosmetics, medicines and other fields.

The present invention provides a peptide derivative represented by Formula (I), or a stereoisomer thereof, or a mixture of stereoisomers thereof,

R₁-Gly-Pro-Gln-Gly-Pro-Gln-R₂ (I)

in Formula (I),
R₁ is selected from a substituent (CA-) or a substituent (GA-), wherein the substituent (CA-) is 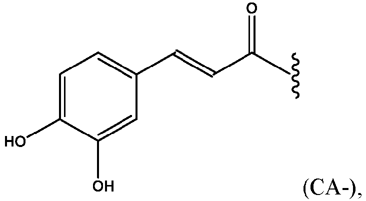
the substituent (GA-) is 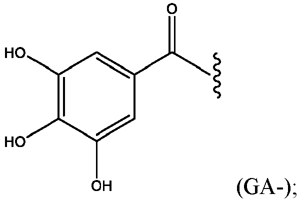
R₂ is selected from -NR₃R₄ or -OR₃, wherein each R₃ and R₄ is independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally is selected from methyl, ethyl, isopropyl, isobutyl, *tert-*butyl*,* pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, optionally has 1, 2, or 3 conjugated or non-conjugated carbon-carbon double bonds; the alkenyl is bonded to the rest portions of a molecule through a single bond, optionally is selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; - NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

Optionally, R₃ and R₄ are each independently selected from H, methyl, ethyl, hexyl, dodecyl or hexadecyl;
optionally, R₃ is H and R₄ is selected from H, methyl, ethyl, hexyl, dodecyl or hexadecyl;
optionally, R₂ is -OH or -NH₂.

A peptide derivative represented by Formula (I), or a stereoisomer thereof, or a mixture of stereoisomers thereof, is selected from the following peptide derivatives (1)-(4):
(1) CA-Gly-Pro-Gln-Gly-Pro-Gln-OH;
(2) CA-Gly-Pro-Gln-Gly-Pro-Gln-NH₂;
(3) GA-Gly-Pro-Gln-Gly-Pro-Gln-OH;
(4) GA-Gly-Pro-Gln-Gly-Pro-Gln-NH₂.

The peptide derivative represented by Formula (I) of the present invention may be present as a stereoisomer thereof, or a mixture of stereoisomers thereof; for example, these amino acids comprised therein may have an L- or D-configuration, or be each independently racemic. Thus, it is possible to obtain isomeric mixtures as well as racemic or diastereomeric mixtures, or pure diastereomers or enantiomers, depending on the number of asymmetric carbons and what isomers or isomeric mixtures are present. Preferred structures of the peptide derivatives represented by Formula (I) of the present invention are pure isomers, i.e., enantiomers or diastereomers.

For example, when -Pro- is mentioned in the present invention, it should be understood that -Pro- is selected from -L-Pro-, -D-Pro-, or a mixture of both, and is racemic or non-racemic. The preparation methods described in this document enable a person skilled in the art to obtain each stereoisomer of the peptide derivative of the present invention by selecting an amino acid having the correct configuration.

The synthesis of the peptide derivative represented by Formula (I) of the present invention or a stereoisomer thereof can be carried out according to conventional methods known in the prior art, such as solid-phase synthesis, liquid-phase synthesis and a method of combining solid-phase and liquid-phase, as well as by biotechnological methods aimed at producing the desired sequence, or by controlling hydrolysis of animal-, fungal-, or plant-derived proteins.

For example, a method for obtaining a peptide derivative represented by Formula (I) comprises the following steps:
- coupling an amino acid having a protected N-terminal and a free C-terminal to an amino acid having a free N-terminal and a C-terminal that is protected or bound to a solid carrier;
- removing the N-terminal protecting group;
- repeating the coupling order and removing the N-terminal protecting group until the desired peptide sequence is obtained; and
- removing the C-terminal protecting group or cleaving from the solid carrier.

Preferably, the C-terminal is bound to a solid carrier and the method is carried out on the solid phase, comprising coupling an amino acid having a protected N-terminal and a free C-terminal to an amino acid having a free N-terminal and a C-terminal that is bound to a polymer carrier; removing the N-terminal protecting group; repeating this order by the required times thereby obtaining a peptide having the desired length, followed by cleaving the synthesized peptide from the initial polymer carrier.

The functional groups of the side chains of the amino acids remain fully protected with temporary or permanent protecting groups throughout the synthesis and can be deprotected simultaneously or orthogonally in the process of cleaving the peptide from the polymer carrier.

Alternatively, solid phase synthesis can be performed by a convergent strategy in which a dipeptide or tripeptide is coupled to a polymer support or to a dipeptide or amino acid previously bound to a polymer support.

The method may comprise additional steps of deprotecting the N-terminal and C-terminal and/or cleaving the peptide from the polymer support in a non-determined order using standard conditions and methods known in the art, and subsequently modifying the terminal functional groups. Optional modifications to N-terminal and C-terminal can be made to the peptide bound to the polymer support, or optional modifications to N-terminal and C-terminal can be made after the peptide has been cleaved from the polymer support.

Another aspect of the present invention provides a cosmetic or pharmaceutical composition, comprising an effective amount of the peptide derivative represented by Formula (I), or a stereoisomer thereof, or a mixture of stereoisomers thereof, and at least one excipient and optionally a cosmetically or pharmaceutically acceptable adjuvant.

Optionally, the adjuvant is selected from a collagen synthesis stimulator, an agent that modulates the PGC-1α synthesis, an agent that modulates the activity of PPARγ, an agent that increases or decreases a triglyceride content in adipocytes, an agent that stimulates or delays adipocyte differentiation, a lipolytic agent or an agent that stimulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinases inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates the HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, a comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, an agent derived from a biological fermentation process, an inorganic salt, a cell extract, a sunscreen, and an effective organic or inorganic photoprotectant against A and/or B UV rays, or mixtures thereof.

Optionally, a preparation of the cosmetic or pharmaceutical composition is selected from a cream, an oil, milk, a balm, a foam, a lotion, a gel, a liniment, a sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or gelatin;
optionally, the capsule includes a soft capsule, a hard capsule, optionally a gelatin capsule;
optionally, the tablet includes a sugar-coated tablet.

The cosmetically or pharmaceutically effective amounts of the peptide derivatives of the present invention to be administered, as well as doses thereof, will depend on many factors including age, the state of a patient, the severity of a disorder or disease, the route and frequency of administration, and specific properties of the peptide derivatives to be used.

"A cosmetically or pharmaceutically effective amount" refers to an amount of one or more peptide derivatives of the present invention that is nontoxic but sufficient to provide the desired effects. The peptide derivative of the present invention is used in the cosmetic or pharmaceutical composition of the present invention at a cosmetically or pharmaceutically effective concentration to obtain the desired effects. In one preferred form, relative to the total weight of the composition, the concentration is between 0.00000001% (by weight) and 20% (by weight), preferably between 0.000001% (by weight) and 15% (by weight), more preferably between 0.0001% (by weight) and 10 % (by weight), and even more preferably between 0.0001% (by weight) and 5% (by weight).

Another aspect of the present invention provides a cosmetically or pharmaceutically acceptable delivery system or sustained release system, comprising an effective amount of the peptide derivative represented by Formula (I), or a stereoisomer thereof, or a mixture of stereoisomers thereof, or the cosmetic or pharmaceutical composition, to achieve better penetration of the active ingredient and/or to improve pharmacokinetic and pharmacodynamic properties thereof.

The term "delivery system" refers to a diluent, adjuvant, excipient or carrier administered with the peptide derivative of the present invention, selected from water, oil or surfactants, including those petroleum-derived, animal-derived, plant-derived, or synthesis-derived. Examples include but are not limited to peanut oil, soybean oil, mineral oil, sesame oil, castor oil, polysorbate, sorbitan ester, ether sulfate, sulfate, betaine, glucoside, maltoside, fatty alcohol, nonoxynol, poloxamer, polyoxyethylene, macrogol, dextrose, glycerin, digitonin and the like. Diluents that can be used in the different delivery systems in which the peptide derivative of the present invention can be administered are known to those of ordinary skill in the art.

The term "sustained release" is used in the conventional sense to refer to a delivery system that provides a gradual release of a compound over a period of time, and preferably, but not necessarily, a relatively constant level of compound release over the entire period of time.

Examples of the delivery system or sustained release system include a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle. The preferred delivery system or sustained release system is a liposome and a microemulsion, more preferably a water-in-oil microemulsion having an internal structure of reverse micelle.

The sustained release system can be prepared by methods known in the art and can be administered, for example, by topical or transdermal administration, including adhesive patches, non-adherent patches, sealing patches, and microelectronic patches; or by systemic administration, such as but not limited to, oral or parenteral routes, including nasal, rectal, subcutaneous implantation or injection, or direct implantation or injection to specific body parts. Preferably a relatively constant amount of these peptide derivatives of the present invention should be released. The amount of a peptide derivative comprised in the sustained release system will depend, for example, on the site where the composition is to be administered, the release kinetics and duration of the peptide derivative of the present invention, and the nature of the condition, disorder and/or disease to be treated and/or cared for.

Another aspect of the present invention provides use of the peptide derivative represented by Formula (I), or a stereoisomer thereof, or a mixture of stereoisomers thereof, or the cosmetic or pharmaceutical composition above, or the cosmetically or pharmaceutically acceptable delivery system or sustained release system above in the preparation of a cosmetic composition or pharmaceutical composition for treating, preventing and/or repairing signs of aging and/or photoaging of the skin.

Another aspect of the present invention provides use of the peptide derivative represented by Formula (I), or a stereoisomer thereof, or a mixture of stereoisomers thereof, or the cosmetic or pharmaceutical composition above, or the cosmetically or pharmaceutically acceptable delivery system or sustained release system above in the preparation of a cosmetic composition or pharmaceutical composition for scavenging free radicals or anti-oxidation.

In order to facilitate understanding of the present invention, the meanings of some terms and expressions used in the present invention are explained as follows.

In the present invention, the term "skin" should be understood to be the layers which comprise it, from the uppermost layer or stratum corneum to the lowermost layer or subcutaneous tissue, both inclusive. These layers are composed of different types of cells such as keratinocytes, fibroblasts, melanocytes and/or adipocytes, among others. In the present invention, the term "skin" includes the scalp.

The term "treatment" means the administration of a peptide derivative according to the present invention to alleviate or eliminate a disease or disorder, or reduce or eliminate one or more symptoms associated with this disease or disorder. The term "treatment" also covers the ability to alleviate or eliminate the physiological consequences of the disease or disorder.

The term "prevention" refers to the ability of a peptide derivative of the present invention to prevent, delay or hinder the appearance or development of a disease or disorder before its appearance.

The term "aging" refers to changes experienced by the skin with age (natural aging) or through exposure to the sun (photoaging) or to environmental contaminants such as chemical fouling or contaminants, tobacco smoke, including all the externally visible and/or perceptible changes through touch, such as but not limited to, the development of discontinuities on the skin (e.g., wrinkles, fine lines, expression lines, stretch lines, striae, furrows, irregularities or roughness, increased pore size, loss of moisture, loss of elasticity, loss of firmness, loss of smoothness, loss of the capacity to recover from deformation, loss of resilience), sagging of the skin (e.g., sagging cheeks, bags under the eyes, or double chins, etc.), changes in skin color (such as scarring, redness, bags under the eyes, or areas of hyperpigmentation like age spots or freckles, etc.), abnormal differentiation, hyperkeratinization, elastosis, keratosis, hair loss, orange peel skin, loss of collagen structure, and other histological changes in the stratum corneum, dermis, epidermis, vasculature (e.g., the appearance of spider veins or telangiectasias), or those tissues adjacent to the skin.

The term "photoaging" refers to premature aging of the skin due to long-term exposure of the skin to UV radiation, exhibiting the same physiological characteristics as natural aging, such as but not limited to, looseness, sagging, color changes or irregularities in the pigmentation, abnormal and/or excessive keratinization.

In the description, the abbreviations used for amino acids follow the rules specified by the IUPAC-IUB Commission of Biochemical Nomenclature in the European Journal of Biochemistry (Eur. J. Biochem. 1984, 138:9-37).

Thus, for example, Gly represents NH₂-CH₂-COOH, Gly- represents NH₂-CH₂-CO-, - Gly represents -NH-CH₂-COOH, and -Gly- represents -NH-CH₂-CO-. Thus, a hyphen representing a peptide bond removes OH in amino acid 1-carboxyl (herein represented in the conventional non-ionized form) when located to the right of the symbol, and removes H in amino acid 2-amino when located to the left of the symbol; both modifications can be applied to the same symbol (see Table 1).

**Table 1 Structures of amino acid residues and one-letter and three-letter abbreviations thereof**

| Name/Abbreviation | Residue | Name/Abbreviation | Residue |
|---|---|---|---|
| Glycyl | 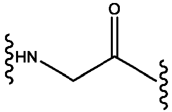 | Prolyl | 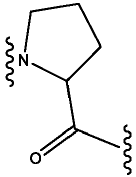 |
| -Gly- | | -Pro- | |
| G | | P | |
| Glutaminyl | 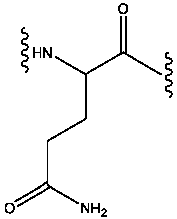 | | |
| -Gln- | | | |
| Q | | | |

The abbreviation "CA-" is used herein to indicate caffeoyl, and the abbreviation "GA- " is used herein to indicate galloyl.

The beneficial effects that the present invention achieves over the prior art include:
1. The peptide derivative of the present invention is obtained through artificial design and is easy to synthesize.
2. 500 ppm caffeic acid has significant toxicity to fibroblasts, gallic acid also has significant toxicity at a concentration of 100 ppm and 500 ppm. After the peptide derivative of the present invention is obtained by covalently binding caffeic acid/gallic acid to the N-terminal of Gly-Pro-Gln-Gly-Pro-Gln, the peptide derivative not only have no toxic effect on fibroblasts but can also promote the proliferation thereof.
3. The peptide derivative of the present invention is a newly structured compound obtained by linking the N-terminal of Gly-Pro-Gln-Gly-Pro-Gln to caffeic acid or gallic acid through an amide bond. Compared with Gly-Pro-Gln-Gly-Pro-Gln, caffeic acid or gallic acid alone, the peptide derivative of the present invention has significantly improved anti-photoaging repair ability and antioxidant effect, achieves unexpected technical effects showing better anti-aging efficacy, and can be used in the fields of medicines, cosmetics, etc.

### BRIEF DESCRIPTION OF THE DRAWINGS

FIG. 1 is a graph showing the effect of the peptide derivatives of the present invention on the proliferation of NIH3T3 cells. * indicates a statistical difference between the drug group and the control group, p<0.05 (n=3). ** indicates a significant difference between the drug group and the control group, p<0.01 (n=3). *** indicates an extremely significant difference between the drug group and the control group, p<0.001 (n=3).
FIG. 2 shows images of the staining morphology of β-galactosidase.
FIG. 3 is a graph showing the statistical results of β-galactosidase staining of senescent cells using the peptide derivative of the present invention at a dose of 10 ppm. ### indicates an extremely significant difference between the UV group and the non-irradiated control group, p<0.001 (n=3). * indicates a statistical difference between the drug group and the control group after UV radiation, p<0.05 (n=3). ** indicates a significant difference between the drug group and the control group after UV radiation, p<0.01 (n=3).
FIG. 4 shows result images indicating the effect of the peptide derivatives of the present invention on fibroblast ROS.

### DETAILED DESCRIPTION OF THE EMBODIMENTS

In order to better understand the present invention, the invention will be described in detail below in conjunction with examples and drawings. However, it should be understood that these examples and drawings are only used for illustration purposes.

### Abbreviations

The abbreviations used for amino acids follow the rules specified by the Biochemical Nomenclature Commission of IUPAC-IUB in Eur. J. Biochem. (1984) 138:9-37 and J. Chem (1989) 264:633-673.

Amide Resin: a starting resin for polypeptide synthesis; Fmoc: 9-fluorenylmethoxycarbonyl;Fmoc-linker:4-[(2,4-dimethoxyphenyl)(Fmoc-amino)methyl]phenoxyacetic acid; DMF: N,N-dimethylformamide; DCM: dichloromethane; DIC: diisopropylcarbodiimide; Ac₂O: acetic anhydride; DIPEA: diisopropylethylamine; piperidine: piperidine; HOBt: 1-hydroxybenzotriazole; TFA: trifluoroacetic acid; TIS: triisopropylsilane; Gly: glycine; Pro: proline; Gln: glutamine; Trt: triphenylmethyl or trityl;
CA: 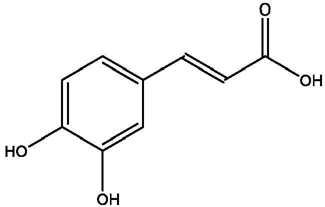 (Caffeic Acid);
"CA-": 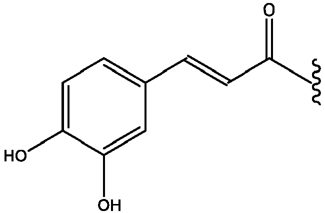 (caffeoyl);
GA: 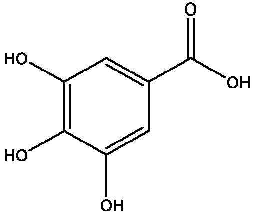 (Gallic Acid) 14
"GA-": 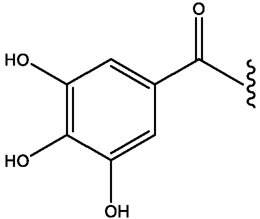 (galloyl).

### EXAMPLE 1

### Preparation of R₁-Gly-Pro-Gln(Trt)-Gly-Pro-Gln(Trt)-Linker-Amide Resin, wherein R₁ is caffeoyl (CA-) or galloyl (GA-).

### 1.1 Preparation of Fmoc-Linker-Amide Resin

20 g Amide Resin having a substitution degree of 1.4 was weighed and added to a solid-phase synthesis reaction column. The resin was washed with 400 ml DMF once and swollen with 500 ml DCM for 30 min. The solvent was removed. The resin was washed with 400 ml DMF three times.

38.4 g (71.1 mmol) Fmoc-linker and HOBt (1.2 eq) were weighed in a dry 500 ml wide-mouth conical flask. After DMF was added for dissolution, the solution was cooled in an ice-water bath for 2 min. DIC (1.5 eq) was added for activation for 3 min while avoiding water vapor. The activated Fmoc-linker was added to the swollen resin to react for 2.5 h, and the reaction solution was removed. The resin was washed with 400 ml DMF three times, each time for 2 min.

400 ml a mixed solution of Ac₂O (5 eq), DIPEA (1 eq), and DMF was added and sealed for 3 h, followed by washing the resin with 500 ml DMF four times and with 500 ml DCM two times. The solvent was removed.

### 1.2 Fmoc deprotection

Fmoc-Linker-Amide Resin was Fmoc-deprotected twice using 500 ml 20% piperidine/DMF, wherein the first reaction lasted 5 min and the second reaction lasted 8 min. The solvent was drained for 3 min. Then 100 ml DMF was added with a nitrogen purge, stirred for 1-2 min, and drained for 3 min. The resin was washed repeatedly 7-8 times. The solvent was removed.

### 1.3 Feeding reaction

21 g Fmoc-Gln(Trt)-OH and 18.2g HOBt were weighed and added into a dry round-bottom flask with ground joint. DMF was added to dissolve the mixture, and the solution was stored in a refrigerator at ± 5 °C. The flask was taken out of the above-mentioned refrigerator 10 min later, followed by adding DIC for activation for 10 min, while avoiding water vapor. The activated amino acid was added to the deprotected resin with a nitrogen purge and stirring. The reaction was carried out at a controlled temperature of 25-35 °C for 2 h. The reaction solution was removed. 100 ml DMF was added with a nitrogen purge and stirring. The resin and the solvent were evenly mixed, then stirred for 1-2 min, and drained for 3 min. The resin was repeatedly washed with 100 ml DMF. The solvent was drained until the filtered solvent was clear and transparent.

The N-terminal Fmoc group was deprotected, and in the presence of 18.2 g HOBt and 21 g DIC, the activated 15.2 g Fmoc-Pro-OH was coupled to a peptidyl resin using DMF as solvent. The reaction lasted 2 h. The resins were then washed and repeatedly subjected to the deprotection treatment of the Fmoc group so as to couple to the next amino acid. In each coupling, in the presence of 18.2 g HOBt and 21 g DIC, and DMF as solvent, the resin was sequentially coupled to 13.4 g Fmoc-Gly-OH; 27.5 g Fmoc-Gln(Trt)-OH; 18.3 g Fmoc-Pro-OH; 16.1 g Fmoc-Gly-OH; and then 8.1 g CA or 7.7 g GA.

After the synthesis, 100 ml DMF was added with a nitrogen purge and stirring. The resin and the solvent were evenly mixed, then stirred for 1-2 min, and drained for 3 min. The resin was repeatedly washed with 100 ml DMF. The solvent was drained until the filtered solvent was clear and transparent.

### 1.4 Shrinking and drying resins

700 ml DCM was added with stirring and nitrogen purge for 3 min and drained for 3 min. 200 ml methanol was added with stirring and nitrogen purge for 5 min and drained for 3 min. 200 ml methanol was added again with stirring and nitrogen purge for 5 min and drained for 30 min. The resins were each poured out to a tray for drying and crushed so that the resins were in a state of quicksand to afford CA-Gly-Pro-Gln(Trt)-Gly-Pro-Gln(Trt)-Linker-Amide Resin and GA-Gly-Pro-Gln(Trt)-Gly-Pro-Gln(Trt)-Linker-Amide Resin, respectively. The resins were weighed, sealed and refrigerated.

### EXAMPLE 2

### Preparation of R₁-Gly-Pro-Gln-Gly-Pro-Gln-NH₂, wherein R₁ is caffeoyl (CA-) or galloyl (GA-).

### 2.1 Cleavage

30 g the dried peptidyl resin obtained in Example 1 was weighed and added to a round-bottom flask. 5.625 ml water, 213.75 ml TFA, and 5.625 ml TIS were taken respectively, mixed evenly, and added to the round-bottom flask containing the peptidyl resin. The mixture was stirred magnetically at 500 rpm for 2.5 h.

After cleavage, the lysate resin was vacuum filtered using a sand core funnel and drained for 1 min. 15 ml TFA was added for washing the resin and drained for 1 min. 15 ml TFA was added again for washing the resin and drained for 1 min. The filtered peptide lysate was obtained.

### 2.2 Sedimentation

2 L frozen isopropyl ether was added into a 5 L serum bottle with stirring. The filtered peptide lysate was slowly added to the 5 L serum bottle, stirred for 5 minutes, and stood for 30 min.

The sedimentation liquid was stratified and yellow-white in color. The supernatant was removed and the lower sediment was poured into two 1 L centrifuge bottles for centrifugation. The centrifugation was performed at a rotation speed of 4000 rpm for 2 min. The supernatant was removed.

The sediment was washed with frozen isopropyl ether. 100 ml isopropyl ether was added to each 1 L centrifuge bottle, stirred for 5 min, and centrifuged at 4000 rpm for 2 min. The supernatant was removed. The washing was repeated four times.

The solid in the centrifuge bottle was blown dry with weak nitrogen for 5 min. The centrifuge bottle was sealed, placed in a vacuum drier, and vacuumed for 2-3 h. The crude CA-Gly-Pro-Gln-Gly-Pro-Gln-NH₂ product and the crude GA-Gly-Pro-Gln-Gly-Pro-Gln-NH₂ product were obtained, respectively.

### EXAMPLE 3

### Preparation and purification using high-performance liquid chromatograph

### 3.1 Pretreatment

11.2 g of the crude CA-Gly-Pro-Gln-Gly-Pro-Gln-NH₂ product was weighed and dissolved in a mixed solvent of 30 ml acetic acid, 30 ml methanol and 300 ml water. The solution was filtered through a 0.22 µm filter membrane for consequent use.

10 g of the crude GA-Gly-Pro-Gln-Gly-Pro-Gln-NH₂ product was weighed and dissolved in a 5% methanol in water. The solution was filtered through a 0.22 µm filter membrane for consequent use.

### 3.2 Purification

High-performance liquid chromatograph was used for preparation and purification.
Mobile phase A: preparative pure acetonitrile; Mobile phase B: 0.1% glacial acetic acid in pure water;
Chromatographic column: Hanbang DAC-100;
Detection wavelength: 215 nm;
Elution gradient of CA-Gly-Pro-Gln-Gly-Pro-Gln-NH₂:

| Time (min) | Flow rate (ml/min) | Mobile phase A (%) | Mobile phase B (%) |
|---|---|---|---|
| 0 | 40 | 5 | 95 |
| 15 | 40 | 10 | 90 |
| 35 | 40 | 30 | 70 |
| 40 | 40 | 35 | 65 |
| 41 | 40 | 70 | 30 |
| 50 | 40 | 70 | 30 |

The filtered sample was purified. Fractions were collected, concentrated and freeze-dried to afford the peptide CA-Gly-Pro-Gln-Gly-Pro-Gln-NH₂ having a purity of 99.12%.

Elution gradient of GA-Gly-Pro-Gln-Gly-Pro-Gln-NH₂:

| Time (min) | Flow rate (ml/min) | Mobile phase A (%) | Mobile phase B (%) |
|---|---|---|---|
| 0 | 15 | 5 | 95 |
| 5 | 15 | 5 | 95 |
| 25 | 15 | 8 | 92 |
| 40 | 15 | 15 | 85 |
| 45 | 15 | 20 | 80 |
| 50 | 15 | 75 | 25 |
| 60 | 15 | 75 | 25 |

The filtered sample was purified. Fractions were collected, concentrated and freeze-dried to afford the peptide GA-Gly-Pro-Gln-Gly-Pro-Gln-NH₂ having a purity of 98.69%.

### EXAMPLE 4

Other compounds of Formula (I) of the present invention can be prepared in similar processes.

The molecular weight of the obtained peptides was determined by ESI-MS. The test results are shown in Table 2 below.

**Table 2 Determination of molecular weight by mass spectrometry**

| No. | Sequence | Structure | Molecular weight |
|---|---|---|---|
| (2) | CA-(Gly-Pro-Gln)₂-NH₂ | 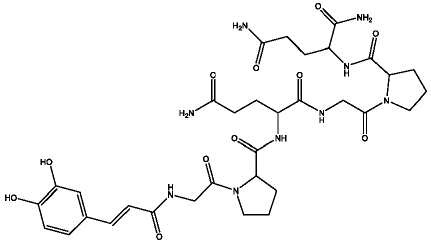 | 743.49 |
| (4) | GA-(Gly-Pro-Gln)₂-NH₂ | 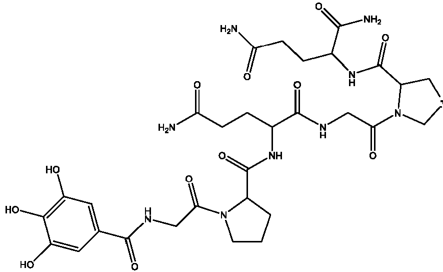 | 733.47 |

### EXAMPLE 5

### Cell proliferation assay

### 5.1 Reagents and materials

Phosphate-buffered saline (PBS) (Gibco), thiazolyl blue (MTT) (Sigma), dimethyl sulfoxide (DMSO) (Sigma), a high glucose culture medium (DMEM) (Gibco), fetal bovine serum (Gibco), CA (caffeic acid) (Solarbio), GA (gallic acid) (Solarbio).

### 5.2 Instruments

Microplate reader (MD, USA), CO₂ incubator (Shanghai Yiheng), ultra-clean workbench (Suzhou Purification).

### 5.3 Cell lines

Mouse skin fibroblasts (NIH3T3) were purchased from the Shanghai Cell Bank of China Center for Type Culture Collection under the Chinese Academy of Sciences.

### 5.4 Samples to be tested

Drug groups:
CA, at a test concentration of 1 ppm, 10 ppm, 100 ppm, and 500 ppm, respectively;
GA, at a test concentration of 1 ppm, 10 ppm, 100 ppm, and 500 ppm, respectively;
the peptide H-Gly-Pro-Gln-Gly-Pro-Gln-NH₂ (hereinafter referred to as the reference peptide), at a test concentration of 1 ppm, 10 ppm, 100 ppm, and 500 ppm, respectively;
the peptide (2) CA-Gly-Pro-Gln-Gly-Pro-Gln-NH₂, at a test concentration of 1 ppm, 10 ppm, 100 ppm, and 500 ppm, respectively;
the peptide (4) GA-Gly-Pro-Gln-Gly-Pro-Gln-NH₂, at a test concentration of 1 ppm, 10 ppm, 100 ppm, and 500 ppm, respectively.
Control group:
   PBS blank control.

### 5.5 Experiment purpose

The purpose of this experiment is to evaluate cell viability 72 h after administration and determine the effect of the peptide derivatives of the present invention on cell proliferation by using NIH3T3 cells as the experiment subject.

### 5.6 Experimental protocol

NIH3T3 cells in good exponential growth phase were taken. A 0.25% trypsin digestion solution was added so that digestion allowed the adherent cells to fall off, counting (1 - 4) × 10⁵ cells/mL, which were prepared into a cell suspension.

The cell suspension was inoculated on a 96-well plate with 200 µL/well, and cultured in a constant-temperature CO₂ incubator for 24 h.

After changing the medium, the cell suspension was added to drug groups and the control group, respectively, each of which was placed in 20 µL/well and incubated in a 37 °C, 5% CO₂ incubator for 72 h.

Then 20 µL 5 mg/ml MTT was added to each well and the incubation was continued for 4 h in the 37°C, 5% CO₂ incubator. The original solution was removed and 150 µL/well DMSO was added. The plate was placed on a plate shaker for shaking for 5 min. A microplate reader was used to determine the OD value of each well at a wavelength of 570 nm, and calculate the cell viability. 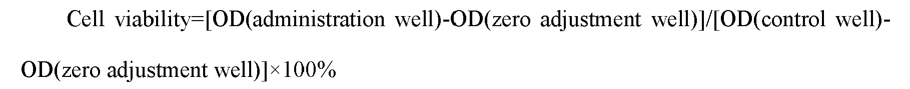 Cell viability=[OD(administration well)-OD(zero adjustment well)]/[OD(control well)-OD(zero adjustment well)] × 100%

### 5.7 Results

The MTT assay is a method of detecting cell survival and growth. The measured OD values are directly proportional to cell viability.

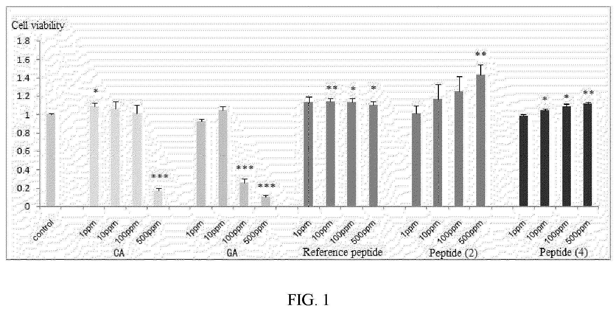

FIG. 1 showed the effect of the peptide derivatives of the present invention on the proliferation of NIH3T3 cells. The results showed that compared with the control group, CA had significant toxicity to NIH3T3 cells at 500 ppm, GA also had significant toxicity at 100 ppm and 500 ppm, and the reference peptide improved cell viability at 10 ppm to 500 ppm.

CA and GA were each linked to the N-terminal of the reference peptide via a covalent bond to obtain newly structured compounds peptide (2) and peptide (4). The peptide (2) could significantly improve cell viability at 500 ppm, and the peptide (4) did not have a toxic effect on NIH3T3 cells at high concentrations. It was hence indicated that after CA and GA were covalently bound to the N-terminal of the reference peptide respectively to obtain the peptide derivatives of the present invention, the toxic effects of the original CA and GA on NIH3T3 cells disappeared. The peptide derivatives of the present invention not only had no toxic effect on fibroblasts, but also could promote the proliferation thereof.

### EXAMPLE 6

### β-Galactosidase staining assay

### 6.1 Reagents and materials

Fetal bovine serum (Gibco), DMEM culture medium (Gibco), penicillin, streptomycin, X-gal (Solarbio), potassium ferricyanide (Solarbio), and potassium ferrocyanide (Solarbio).

### 6.2 Instruments

CO₂ incubator, ultra-clean workbench, and optical microscope.

### 6.3 Cell lines

Mouse skin fibroblasts (NIH3T3) were purchased from the Shanghai Cell Bank of China Center for Type Culture Collection under the Chinese Academy of Sciences.

### 6.4 Samples to be tested

Drug groups:
CA, at a test concentration of 1 ppm, 10 ppm, and 100 ppm, respectively;
GA, at a test concentration of 10 ppm;
the reference peptide, at a test concentration of 1 ppm, 10 ppm, and 100 ppm, respectively;
the peptide (2), at a test concentration of 1 ppm, 10 ppm, and 100 ppm, respectively;
the peptide (4) at a test concentration of 10 ppm.
Control group:
   PBS blank control.

### 6.5 Experiment purpose

The purpose of this experiment is to test the impact of the peptide derivatives of the present invention on cell senescence using a β-galactosidase staining assay to evaluate the anti-photoaging capability.

### 6.6 Experimental protocol

The frozen NIH3T3 cells were cultured and passaged at a ratio of 1:2 to about the 5th generation, and the cells in better growth were selected as experiment subjects.

The cells were inoculated into a 96-well plate at 10,000 cells/well, and when the cells were approximately 80% full, a UV photoaging model was established. In the control group, a PBS solution was added, supplemented with the culture medium to 200 µL, while no UV irradiation was performed; For the UV group and the drug group, a proper amount of PBS was added to wash the culture repeatedly until colorless, followed by adding 50 µL PBS and irradiating under an 80 J/cm³ UV light. The distance between the light source and the culture bottle was 15 cm. After irradiation, the PBS was removed. In the UV group, a PBS solution and the culture medium were added to 200 µL; in the drug group, the culture medium and the drug diluted in a certain ratio were added to 200 µL. The control group, the UV group, and the drug group were further incubated in a 37°C, 5% CO₂ incubator for 24 h.

The culture medium was removed. 100 µL 4% paraformaldehyde was added to each well and fixed at room temperature for 15 min, followed by washing with PBS three times, adding a β-galactosidase staining agent (X-gal 0.01%, potassium ferricyanide 0.16%, ferrocyanide potassium 0.02%) 50 µL/well, sealing with plastic wrap, and staining in a 37°C incubator in the dark for 12 h. The staining solution was removed. Each group was washed with PBS three times, and photographed under a light microscope. The number of cells in the field of view and the number of stained cells were counted.

### 6.7 Results

UV irradiation can cause cell senescence, and the cytoplasm of senescent cells are stained blue-green by the β-galactosidase agent (shown as the dark part in the black and white images). This experiment evaluates the anti-photoaging capability of the peptide derivatives of the present invention through staining morphology images and senescent cell staining statistics.

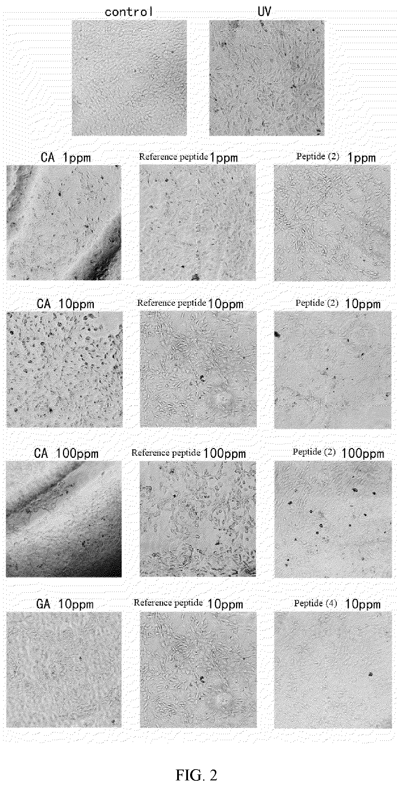

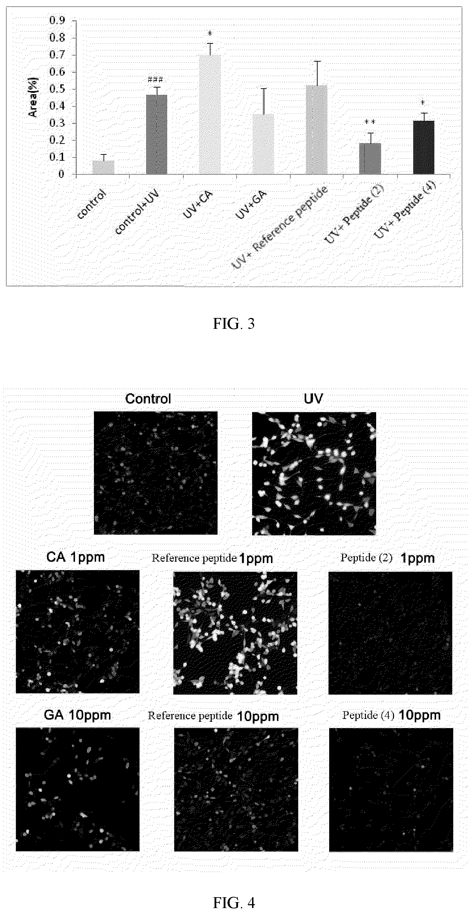

FIGS. 2 and 3 were images showing β-galactosidase staining morphology and a graph showing the statistical results of β-galactosidase staining of senescent cells by the peptide derivatives of the present invention at a dose of 10 ppm, respectively. The results showed that after UV irradiation, senescent cells increased significantly (shown in the dark part in FIG 2); after administration, CA was unable to reduce the proportion of senescent cells at the test concentrations, and even more senescent cells were observed at 10 ppm; GA and the reference peptide were also unable to reduce the proportion of senescent cells. It was demonstrated that none of CA, GA, and the reference peptide could improve cell photoaging. In contrast, the peptide (2) of the present invention significantly reduced the proportion of senescent cells at a concentration of 1 ppm to 100 ppm, and the peptide (4) also reduced the proportion of senescent cells at a concentration of 10 ppm. It was hence indicated that the newly structured compounds obtained by linking the N-terminal of the reference peptide to CA or GA through an amide bond showed unexpected anti-photoaging repair ability compared to the reference peptide, CA or GA alone.

### EXAMPLE 7

### ROS staining assay

### 7.1 Reagents and materials

Fetal bovine serum, DMEM culture medium, penicillin, and streptomycin.

### 7.2 Instruments

CO₂ incubator, ultra-clean workbench, and fluorescence microscope.

### 7.3 Cell lines

Mouse skin fibroblasts (NIH3T3) were purchased from the Shanghai Cell Bank of China Center for Type Culture Collection under the Chinese Academy of Sciences.

### 7.4 Samples to be tested

Drug groups:
CA, at a test concentration of 1 ppm;
GA, at a test concentration of 10 ppm;
the reference peptide, at a test concentration of 1 ppm and 10 ppm, respectively;
the peptide (2), at a test concentration of 1 ppm;
the peptide (4), at a test concentration of 10 ppm.
Control group:
   PBS blank control.

### 7.5 Experiment purpose

Another indication of aging is an increase in intracellular oxidative stress levels, that is, an increase in ROS, and aging can be slowed down by alleviating oxidative stress. The purpose of this experiment is to test the impact of the peptide derivatives of the present invention on oxidative stress levels using ROS staining assay, thereby evaluating the antioxidant effect.

### 7.6 Experimental protocol

The frozen NIH3T3 cells were cultured and passaged at a ratio of 1:2 to about the 5th generation, and the cells in better growth were selected as experiment subjects.

The cells were inoculated into a 96-well plate at 10,000 cells/well, and when the cells were approximately 80% full, a UV photoaging model was established. In the control group, a PBS solution was added, supplemented with the culture medium to 200 µL, while no UV irradiation was performed; For the UV group and the drug group, a proper amount of PBS was added to wash the culture repeatedly until colorless, followed by adding 50 µL PBS and irradiating under an 80 J/cm³ UV light. The distance between the light source and the culture bottle was 15 cm. After irradiation, the PBS was removed. In the UV group, a PBS solution and the culture medium were added to 200 µL; in the drug group, the culture medium and the drug diluted in a certain ratio were added to 200 µL. The control group, the UV group, and the drug group were further incubated in a 37°C, 5% CO₂ incubator for 24 h.

The culture medium was removed. Each group was stained according to the ROS probe instructions for 20 min and was observed under a fluorescence microscope.

### 7.7 Results

Based on the UV photoaging model, a ROS fluorescent probe was used for labeling to evaluate the antioxidant capacity of the peptide derivatives of the present invention. The results were shown in FIG. 4. After UV irradiation, the cell density decreased, and the expression of ROS increased in cells of the same unit (shown in the light color part of FIG. 4). The reference peptide at 1 ppm failed to reduce the ROS level per unit cells; when at 1ppm, CA reduced the ROS level per unit cells to a certain extent. In contrast, the peptide (2) at 1 ppm significantly reduced the expression of ROS per unit cells and had excellent antioxidant capacity. On the other hand, both the reference peptide and GA had a certain effect in reducing ROS at 10 ppm; but the peptide (4) at 10 ppm more significantly reduced the expression of ROS per unit cells. It was hence indicated that the newly structured compounds obtained by linking the N-terminal of the reference peptide to CA or GA through an amide bond showed unexpected antioxidant effects compared to the reference peptide, CA or GA alone.

Compared with the reference peptide, caffeic acid or gallic acid alone, the peptide derivatives of the present invention had significantly improved anti-photoaging repair ability and antioxidant effect, achieved unexpected technical effects showing better anti-aging efficacy, and could be used in the fields of medicines, cosmetics, etc.

### EXAMPLE 8

### Preparation of a skin toner comprising the peptide (2)

| Ingredients | by weight % |
|---|---|
| Propylene glycol | 1.0 |
| Allantoin | 0.3 |
| Glycerin | 1.0 |
| PEG-7 glyceryl cocoate | 1.0 |
| Peptide (2) | 0.005 |
| Preservative | 0.5 |
| Perfume | 0.5 |
| Water | Balance to 100 |

Allantoin and glycerin were dissolved in water and heated to 85 °C at which the temperature was kept for 30 min; PEG-7 glyceryl cocoate and the peptide (2) were dissolved in water; the above solutions were mixed after cooling, and stirred evenly to afford a mixed solution; Propylene glycol, a preservative, and perfume were added to the above mixed solution in turn, and water was added with stirring evenly to afford a skin toner.

### EXAMPLE 9

### Preparation of a cleansing gel comprising the peptide (2)

| Ingredients | by weight % |
|---|---|
| Carbomer (2% lotion) | 25.0 |
| Triethanolamine | 0.5 |
| Peptide (2) | 0.005 |
| Preservative | 0.2 |
| EDTA | 0.1 |
| Perfume | 0.5 |
| Water | Balance to 100 |

The peptide (2) was dissolved in water and stirred evenly to afford a polypeptide solution; carbomer and EDTA were dissolved in water and heated to 85 °C; the temperature was kept for 30 min; after cooling triethanolamine was added and stirred evenly to afford a mixed solution; the above mixed solution, the polypeptide solution, a preservative, and perfume were added to water in turn and stirred until completely swollen to afford a cleansing gel.

### EXAMPLE 10

An emulsion composition comprising the peptide (4)

| Ingredients | by weight % |
|---|---|
| A cetearyl alcohol (and) cetearyl glucoside | 5.0 |
| Jojoba oil | 5.0 |
| Mineral oil | 5.0 |
| Isopropyl palmitate | 7.0 |
| B Glycerin | 5.0 |
| Allantoin | 0.1 |
| Polyacrylamide (and) C13-14 isoparaffin (and) laureth-7 | 0.3 |
| Water | Balance to 100 |
| C Preservative | 0.5 |
| D perfume | 0.5 |
| Peptide (4) | 0.01 |

0.01 g the peptide (4) was formulated into a 0.02 mg/mL aqueous solution; cetearyl alcohol (and) cetearyl glucoside, jojoba oil, mineral oil, and isopropyl palmitate were heated to 85 °C, and stirred evenly to afford phase A; glycerin, allantoin, polyacrylamide (and) C13-14 isoparaffin (and) laureth-7 were dissolved in water, and heated to 85 °C to afford phase B; phase A was quickly added into phase B, homogenized at a constant temperature for 3-5 min and cooled; when the above mixture was cooled to below 60 °C, a preservative was added and stirred evenly; when cooled to below 45°C, a polypeptide solution and perfume were added to afford an emulsion.

## Claims

1. A peptide derivative represented by Formula (I), or a stereoisomer thereof, or a mixture of stereoisomers thereof,
R₁-Gly-Pro-Gln-Gly-Pro-Gln-R₂ (I)
in Formula (I),
R₁ is selected from a substituent (CA-) or a substituent (GA-), wherein the substituent (CA-) is 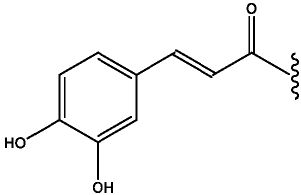
(CA-),
the substituent (GA-) is 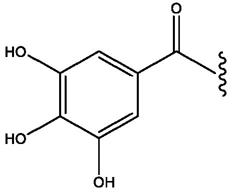 (GA-);
R₂ is selected from -NR₃R₄ or -OR₃, wherein each R₃ and R₄ is independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally is selected from methyl, ethyl, isopropyl, isobutyl, *tert-*butyl*,* pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, optionally has 1, 2, or 3 conjugated or non-conjugated carbon-carbon double bonds; the alkenyl is bonded to the rest portions of a molecule through a single bond, optionally is selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; - NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

2. The peptide derivative represented by Formula (I) according to claim 1, or a stereoisomer thereof, or a mixture of stereoisomers thereof, **characterized in that** R₃ and R₄ are independently selected from H, methyl, ethyl, hexyl, dodecyl or hexadecyl;
optionally, R₃ is H and R₄ is selected from H, methyl, ethyl, hexyl, dodecyl or hexadecyl;
optionally, R₂ is -OH or -NH₂.

3. The peptide derivative represented by Formula (I) according to any of claims 1-2, or a stereoisomer thereof, or a mixture of stereoisomers thereof, **characterized in that** it is selected from the following peptide derivatives (1)-(4):
(1) CA-Gly-Pro-Gln-Gly-Pro-Gln-OH;
(2) CA-Gly-Pro-Gln-Gly-Pro-Gln-NH₂;
(3) GA-Gly-Pro-Gln-Gly-Pro-Gln-OH;
(4) GA-Gly-Pro-Gln-Gly-Pro-Gln-NH₂

4. A cosmetic or pharmaceutical composition, **characterized in that** it includes an effective amount of the peptide derivative represented by Formula (I) according to any of claims 1-3, or a stereoisomer thereof, or a mixture of stereoisomers thereof, and at least one excipient and optionally a cosmetically or pharmaceutically acceptable adjuvant;
optionally, the adjuvant is selected from a collagen synthesis stimulator, an agent that modulates the PGC-1α synthesis, an agent that modulates the activity of PPARγ, an agent that increases or decreases a triglyceride content in adipocytes, an agent that stimulates or delays adipocyte differentiation, a lipolytic agent or an agent that stimulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinases inhibitor, a melanin synthesis stimulator or inhibitor, a whitening or decoloring agent, a pigmentation promoter, a self-tanning agent, an antiaging agent, a NO-synthase inhibitor, a 5 α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates the HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, a comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, an agent derived from a biological fermentation process, an inorganic salt, a cell extract, a sunscreen, and an effective organic or inorganic photoprotectant against A and/or B UV rays, or mixtures thereof.

5. The cosmetic or pharmaceutical composition according to claim 4, **characterized in that** a preparation of the cosmetic or pharmaceutical composition is selected from a cream, an oil, milk, a balm, a foam, a lotion, a gel, a liniment, a sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or gelatin;
optionally, the capsule includes a soft capsule, a hard capsule, optionally a gelatin capsule; optionally, the tablet includes a sugar-coated tablet.

6. A cosmetically or pharmaceutically acceptable delivery system or sustained release system, **characterized in that** it comprises an effective amount of the peptide derivative represented by Formula (I) according to any of claims 1-3, or a stereoisomer thereof, or a mixture of stereoisomers thereof, or the cosmetic or pharmaceutical composition according to claim 4 or 5;
the cosmetically or pharmaceutically acceptable delivery system or sustained release system is selected from a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle; or optionally a liposome or microemulsion; or optionally a water-in-oil microemulsion having an internal structure of reverse micelle.

7. Use of the peptide derivative represented by Formula (I) according to any of claims 1-3, or a stereoisomer thereof, or a mixture of stereoisomers thereof, or the cosmetic or pharmaceutical composition according to claim 4 or 5, or the cosmetically or pharmaceutically acceptable delivery system or sustained release system according to claim 6 in the preparation of a cosmetic composition or pharmaceutical composition for treating, preventing and/or repairing signs of aging and/or photoaging of the skin.

8. Use of the peptide derivative represented by Formula (I) according to any of claims 1-3, or a stereoisomer thereof, or a mixture of stereoisomers thereof, or the cosmetic or pharmaceutical composition according to claim 4 or 5, or the cosmetically or pharmaceutically acceptable delivery system or sustained release system according to claim 6 in the preparation of a cosmetic composition or pharmaceutical composition for non-therapeutically scavenging free radicals or anti-oxidation.

## Patentansprüche

1. Peptidderivat, dargestellt durch Formel (I), oder ein Stereoisomer davon oder eine Mischung von Stereoisomeren davon,
R₁-Gly-Pro-Gln-Gly-Pro-Gln-R₂ (I)
wobei in Formel (I)
R₁ ausgewählt ist aus einem Substituenten (CA-) oder einem Substituenten (GA-), wobei der Substituent (CA-) 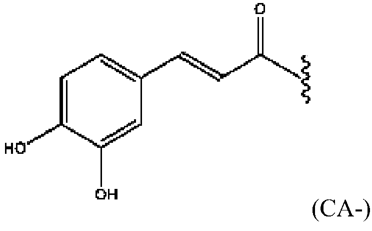 ist,
der Substituent (GA-) 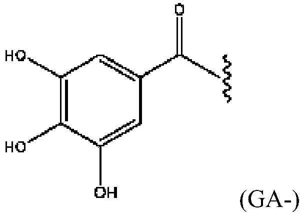 ist;
R₂ ausgewählt ist aus -NR₃R₄ oder -OR₃, wobei R₃ und R₄ jeweils unabhängig ausgewählt sind aus H, substituiertem oder unsubstituiertem Alkyl, substituiertem oder unsubstituiertem Alkenyl;
wobei sich das Alkyl auf ein gesättigtes aliphatisches lineares oder verzweigtes Alkyl mit 1-24 Kohlenstoffatomen bezieht (wahlweise mit 1-16 Kohlenstoffatomen; wahlweise mit 1-14 Kohlenstoffatomen; wahlweise mit 1-12 Kohlenstoffatomen; oder wahlweise mit 1, 2, 3, 4, 5 oder 6 Kohlenstoffatomen); wahlweise ausgewählt ist aus Methyl, Ethyl, Isopropyl, Isobutyl, *tert*-Butyl, Pentyl, Hexyl, Heptyl, Octyl, Decyl, Dodecyl, Tetradecyl, Hexadecyl, Octadecyl, 2-Ethylhexyl, 2-Methylbutyl oder 5-Methylhexyl;
wobei das Alkenyl auf ein lineares oder verzweigtes Alkenyl mit 2-24 Kohlenstoffatomen bezieht (wahlweise mit 2-16 Kohlenstoffatomen; wahlweise mit 2-14 Kohlenstoffatomen; wahlweise mit 2-12 Kohlenstoffatomen; wahlweise mit 2, 3, 4, 5 oder 6 Kohlenstoffatomen); wobei das Alkenyl eine oder mehrere Kohlenstoff-Kohlenstoff-Doppelbindungen aufweist, wahlweise 1, 2 oder 3 konjugierte oder nicht konjugierte Kohlenstoff-Kohlenstoff-Doppelbindungen aufweist; wobei das Alkenyl durch eine Einzelbindung an die Reste eines Moleküls gebunden, und wahlweise ausgewählt ist aus Vinyl, Oleyl oder Linoleyl;
wobei der Substituent in dem "substituierten Alkyl" oder "substituierten Alkenyl" wahlweise ausgewählt ist aus C₁-C₄-Alkyl; Hydroxyl; C₁-C₄-Alkoxy; Amino; C₁-C₄-Aminoalkyl; C₁-C₄-Carbonyloxy; C₁-C₄-Oxycarbonyl; Halogen (wie etwa Fluor, Chlor, Brom und Jod); Cyano; Nitro; Azid; C₁-C₄-Alkylsulfonyl; Thiol; C₁-C₄-Alkylthio; C₆-C₃₀-Aryloxy wie etwa Phenoxy; - NR_{b}(C=NR_{b})NR_{b}R_{c}, wobei R_{b} und R_{c} unabhängig voneinander ausgewählt sind aus H, C₁-C₄-Alkyl, C₂-C₄-Alkenyl, C₂-C₄-Alkynyl, C₃-C₁₀-Cycloalkyl, C₆-C₁₈-Aryl, C₇-C₁₇-Aralkyl, einer heterocyclischen Gruppe mit 3 bis 10 Gliedern oder einer Aminoschutzgruppe.

2. Peptidderivat, dargestellt durch Formel (I), nach Anspruch 1 oder ein Stereoisomer davon oder eine Mischung von Stereoisomeren davon, **dadurch gekennzeichnet, dass** R₃ und R₄ unabhängig voneinander ausgewählt sind aus H, Methyl, Ethyl, Hexyl, Dodecyl oder Hexadecyl;
wobei wahlweise R₃ H ist und R₄ ausgewählt ist aus H, Methyl, Ethyl, Hexyl, Dodecyl oder Hexadecyl;
wobei wahlweise R₂ -OH oder -NH₂ ist.

3. Peptidderivat, dargestellt durch Formel (I) nach einem der Ansprüche 1-2 oder ein Stereoisomer davon oder eine Mischung von Stereoisomeren davon, **dadurch gekennzeichnet, dass** es ausgewählt ist aus den folgenden Peptidderivaten (1)-(4):
(1) CA-Gly-Pro-Gln-Gly-Pro-Gln-OH;
(2) CA-Gly-Pro-Gln-Gly-Pro-Gln-NH₂;
(3) GA-Gly-Pro-Gln-Gly-Pro-Gln-OH;
(4) GA-Gly-Pro-Gln-Gly-Pro-Gln-NH_{2.}

4. Kosmetische oder pharmazeutische Zusammensetzung, **dadurch gekennzeichnet, dass** sie eine wirksame Menge des Peptidderivats, dargestellt durch Formel (I), nach einem der Ansprüche 1 bis 3 oder eines Stereoisomers davon oder einer Mischung von Stereoisomeren davon, und wenigstens einen Träger und wahlweise einen kosmetisch oder pharmazeutisch akzeptablen Hilfsstoff;
wobei der Hilfsstoff wahlweise ausgewählt ist aus einem Kollagensynthesestimulator, einem Mittel, das die PGC-1α-Synthese moduliert, einem Mittel, das die PPARγ-Aktivität moduliert, einem Mittel, das einen Triglyceridgehalt in Adipozyten erhöht oder senkt, einem Mittel, das die Adipozytendifferenzierung stimuliert oder verzögert, einem lipolytischen Mittel oder einem Mittel, das die Lipolyse stimuliert, einem Fettabbaumittel, einem adipogenen Mittel, einem Inhibitor gegen Acetylcholin-Rezeptor-Aggregation, einem Mittel, das die Muskelkontraktion hemmt, einem anticholinergen Mittel, einem Elastaseinhibitor, einem Matrix-Metalloproteinase-Inhibitor, einem Melaninsynthesestimulator oder -inhibitor, einem Weißmacher oder Entfärbungsmittel, einem Pigmentierungsförderer, einem Selbstbräunungsmittel, einem Anti-Aging-Mittel, einem NO-Synthase-Inhibitor, einem 5α-Reduktase-Inhibitor, einem Inhibitor für Lysylhydroxylase und/oder Prolylhydroxylase, einem Antioxidationsmittel, einem Scavenger für freie Radikale und/oder einem Anti-Luftverschmutzungsmittel, einem Scavenger für reaktive Carbonylspezies, einem Antiverzuckerungsmittel, einem Antihistamin, einem antiviralen Mittel, einem antiparasitischen Mittel, einem Emulgator, einem Emolliens, einem organischen Lösungsmittel, einem flüssigen Treibmittel, einem Haut-Conditioner, einem Befeuchtungsmittel, einer Feuchtigkeitsbewahrungssubstanz, einer Alpha-Hydroxy-Säure, einer Beta-Hydroxy-Säure, einem Feuchtigkeitsspender, einer Epidermalhydrolase, einem Vitamin, einer Aminosäure, einem Protein, einem Pigment oder Färbemittel, einem Farbstoff, einem Biopolymer, einem Gelierungspolymer, einem Verdickungsmittel, einem Tensid, einem Weichmacher, einem Klebstoff, einem Konservierungsstoff, einem Anti-Falten-Mittel, einem Mittel zur Reduzierung oder Behandlung von Unter-Augen-Schwellungen, einem Exfoliant, einem Mittel zur Cuticulenentfernung, einem keratolytischen Mittel, einem antimikrobiellen Mittel, einem Antimykotikum, einem Fungistat, einem Bakterizid, einem Bakteriostat, einem Mittel, das die Synthese von dermalen oder epidermalen Makromolekülen stimuliert und/oder deren Abbau hemmt oder verhindert, einem Mittel, das die Elastinsynthese stimuliert, einem Mittel, das die Decorinsynthese stimuliert, einem Mittel, das die Lamininsynthese stimuliert, einem Mittel, das die Defensinsynthese stimuliert, einem Mittel, das die Chaperonproteinsynthese stimuliert, einem Mittel, das die cAMP-Synthese stimuliert, einem Hitzeschockprotein, einem Mittel, das die HSP70-Synthese stimuliert, einem Mittel, das die Synthese eines Hitzeschockproteins stimuliert, einem Mittel, das die Synthese von Hyaluronsäure stimuliert, einem Mittel, das die Fibronectinsynthese stimuliert, einem Mittel, das die Deacetylasesynthese stimuliert, einem Mittel, das die Synthese eines Lipids und einer Stratum-corneum-Komponente stimuliert, Ceramid, einer Fettsäure, einem Mittel, das die Kollagenzersetzung hemmt, einem Mittel, das die Elastinzersetzung hemmt, einem Mittel, das Serinprotease hemmt, einem Mittel, das die Fibroblastenproliferation stimuliert, einem Mittel, das die Keratinozytenproliferation stimuliert, einem Mittel, das die Adipozytenproliferation stimuliert, einem Mittel, das die Melanozytenproliferation stimuliert, einem Mittel, das die Keratinozytendifferenzierung stimuliert, einem Mittel, das Acetylcholinesterase hemmt, einem Hautentspannungsmittel, einem Mittel, das die Glycosaminoglycansynthese stimuliert, einem Anti-Hyperkeratosemittel, einem komedolytischen Mittel, einem Anti-Psoriasis-Mittel, einem Anti-Dermatitis-Mittel, einem Anti-Ekzem-Mittel, einem DNA-Reparaturmittel, einem DNA-Schutzmittel, einem Stabilisator, einem antipruritischen Mittel, einem Mittel zur Behandlung und/oder Pflege empfindlicher Haut, einem Härtungsmittel, einem Straffungsmittel, einem Restrukturierungsmittel, einem Anti-Dehnungsmittel, einem Klebstoff, einem Mittel, das die Talgproduktion moduliert, einem Schweißhemmungsmittel, einem Mittel, das die Heilung stimuliert, einem Mittel, das die Heilung unterstützt, einem Mittel, das die Reepithelialisierung stimuliert, einem Mittel, das die Reepithelialisierung unterstützt, einem Zytokin-Wachstumsfaktor, einem Beruhigungsmittel, einem entzündungshemmenden Mittel, einem Betäubungsmittel, einem Mittel, das auf die Kapillarzirkulation und/oder Mikrozirkulation einwirkt, einem Mittel, das die Angiogenese stimuliert, einem Mittel, das die Gefäßdurchlässigkeit hemmt, einem Venotonikum, einem Mittel, das auf den Zellmetabolismus einwirkt, einem Mittel zum Verbessern der Dermis-Epidermis-Verbindung, einem Mittel, das das Haarwachstum anregt, einem Mittel, das das Haarwachstum hemmt oder verzögert, einem Duftstoff, einem Chelatbildner, einem Pflanzenextrakt, einem essenziellen Öl, einem Meeresextrakt, einem aus einem biologischen Fermentierungsprozess hergeleiteten Mittel, einem anorganischen Salz, einem Zellextrakt, einem Sonnenschutzmittel und einem effektiven organischen oder anorganischen Lichtschutzmittel gegen UV-A- und/oder -B-Strahlen oder Mischungen davon.

5. Kosmetische oder pharmazeutische Zusammensetzung nach Anspruch 4, **dadurch gekennzeichnet, dass** eine Zubereitung der kosmetischen oder pharmazeutischen Zusammensetzung ausgewählt ist aus einer Creme, einem Öl, Milch, einem Balsam, einem Schaum, einer Lotion, einem Gel, einem Einreibemittel, Sera, einer Seife, einem Shampoo, einer Haarspülung, einem Serum, einer Salbe, einem Mousse, einer Pomade, einem Pulver, einem festen Stück, einem Stift, einem Spray, einem Aerosol, einer Kapsel, einer Tablette, einem Granulat, einem Kaugummi, einer Lösung, einer Suspension, einer Emulsion, einem Sirup, einem Elixier, einem Polysaccharidfilm, einem Gallert oder Gelatine;
wobei die Kapsel wahlweise eine Weichkapsel, eine Hartkapsel, wahlweise eine Gelatinekapsel, beinhaltet;
wobei die Tablette wahlweise eine zuckerbeschichtete Tablette beinhaltet.

6. Kosmetisch oder pharmazeutisch akzeptables Abgabesystem oder System zur retardierten Freigabe, **dadurch gekennzeichnet, dass** es eine wirksame Menge des Peptidderivats, dargestellt durch Formel (I), nach einem der Ansprüche 1 bis 3 oder ein Stereoisomer davon oder eine Mischung von Stereoisomeren davon oder die kosmetische oder pharmazeutische Zusammensetzung nach Anspruch 4 oder 5 umfasst;
wobei das kosmetisch oder pharmazeutisch akzeptable Abgabesystem oder System zur retardierten Freigabe ausgewählt ist aus einem Liposom, einem Oleosom, einem nichtionischen Tensid-Liposomvesikel, einem Ethosom, einer Millikapsel, einer Mikrokapsel, einer Nanokapsel, einem nanostrukturierten Lipidträger, einem Schwamm, einem Cyclodextrin, einem Lipoidvesikel, einem Mizell, einer Millikugel, einer Mikrokugel, einer Nanokugel, einer Lipokugel, einer Mikroemulsion, einer Nanoemulsion, einem Millipartikel, einem Mikropartikel oder einem Nanopartikel; oder wahlweise einem Liposom oder einer Mikroemulsion; oder wahlweise einer Wasser-in-Öl-Mikroemulsion mit einer internen Struktur eines Reversmizells.

7. Verwendung des Peptidderivats, dargestellt durch Formel (I), nach einem der Ansprüche 1 bis 3 oder eines Stereoisomers davon oder einer Mischung von Stereoisomeren davon oder der kosmetischen oder pharmazeutischen Zusammensetzung nach Anspruch 4 oder 5 oder des kosmetisch oder pharmazeutisch akzeptablen Abgabesystems oder Systems zur retardierten Freigabe nach Anspruch 6 bei der Herstellung einer kosmetischen Zusammensetzung oder pharmazeutischen Zusammensetzung zur Behandlung, Prävention und/oder Reparatur von Zeichens der Alterung und/oder Lichtalterung der Haut.

8. Verwendung des Peptidderivats, dargestellt durch Formel (I), nach einem der Ansprüche 1 bis 3 oder eines Stereoisomers davon oder einer Mischung von Stereoisomeren davon oder der kosmetischen oder pharmazeutischen Zusammensetzung nach Anspruch 4 oder 5 oder des kosmetisch oder pharmazeutisch akzeptablen Abgabesystems oder Systems zur retardierten Freigabe nach Anspruch 6 bei der Herstellung einer kosmetischen Zusammensetzung oder pharmazeutischen Zusammensetzung zum nicht-therapeutischen Auffangen von freien Radikalen oder zur Antioxidation.

## Revendications

1. Un dérivé peptidique représenté par la Formule (I), ou un stéréoisomère de celui-ci, ou un mélange de stéréoisomères de celui-ci,
R₁ -Gly-Pro-Gln-Gly-Pro-Gln-R₂ (I)
dans la Formule (I),
R₁ est sélectionné parmi un substituant (CA-) ou un substituant (GA-), dans lequel le substituant (CA-) est 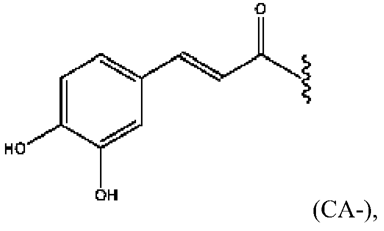 le substituant (GA-) est 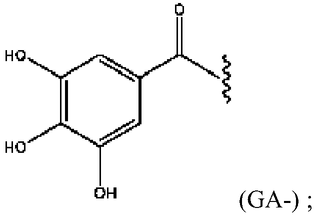
R₂ est sélectionné parmi -NR₃R₄ ou -OR₃, où chacun de R₃ et R₄ est indépendamment choisi parmi H, alkyle substitué ou non-substitué, alcényle substitué ou non-substitué ;
l'alkyle fait référence à un alkyle en chaîne linéaire ou ramifiée aliphatique saturée ayant 1 à 24 atomes de carbone (ayant optionnellement 1 à 16 atomes de carbone ; ayant optionnellement 1 à 14 atomes de carbone ; ayant optionnellement 1 à 12 atomes de carbone ; ou ayant optionnellement 1, 2, 3, 4, 5 ou 6 atomes de carbone) ; optionnellement est choisi parmi méthyle, éthyle, isopropyle, isobutyle, *tert*-butyle, pentyle, hexyle, heptyle, octyle, décyle, dodécyle, tétradécyle, hexadécyle, octadécyle, 2-éthylhexyle, 2-méthylbutyle ou 5-méthylhexyle ;
l'alcényle fait référence à un alcényle en chaîne linéaire ou ramifiée ayant 2 à 24 atomes de carbone (ayant optionnellement 2 à 16 atomes de carbone ; ayant optionnellement 2 à 14 atomes de carbone ; ayant optionnellement 2 à 12 atomes de carbone ; ayant optionnellement 2, 3, 4, 5 ou 6 atomes de carbone) ; l'alcényle possède une ou plusieurs doubles liaisons carbone-carbone, optionnellement 1, 2 ou 3 doubles liaisons carbone-carbone conjuguées ou non conjuguées ; l'alcényle est lié aux parties restantes d'une molécule par une liaison simple, étant optionnellement choisi parmi vinyle, oléyle ou linoléyle ;
optionnellement, le substituant dans « l'alkyle substitué » ou « l'alcényle substitué » est sélectionné parmi l'alkyle C₁-C₄ ; hydroxyle ; alcoxy C₁-C₄ ; amino ; aminoalkyle C₁-C₄ ; carbonyloxy C₁-C₄ ; oxycarbonyle C₁-C₄ ; halogène (comme le fluor, le chlore, le brome et l'iode) ; cyano ; nitro ; azoture ; alkylsulfonyle C₁-C₄ ; thiol ; alkylthio C₁-C₄ ; aryloxy C₆-C₃₀ tel que le phénoxy ; - NR_{b}(C=NR_{b})NR_{b}R_{c}, où R_{b} et R_{c} sont sélectionnés indépendamment parmi H, alkyle C₁-C₄ , alcényle C₂-C₄, alcynyle C₂-C₄, cycloalkyle C₃-C₁₀, C₆-C₁₈ aryle, C₇-C₁₇ aralkyle, un groupe hétérocyclique de 3 à 10 chaînons, ou un groupe de protection des aminés.

2. Le dérivé peptidique représenté par la Formule (I) selon la revendication 1, ou un stéréoisomère de celui-ci, ou un mélange de stéréoisomères de celui-ci, **caractérisé en ce que** R₃ et R₄ sont indépendamment choisis parmi H, méthyle, éthyle, hexyle, dodécyle ou hexadécyle ;
optionnellement, R₃ est H et R₄ est sélectionné parmi H, méthyle, éthyle, hexyle, dodécyle ou hexadécyle ;
optionnellement, R₂ est -OH ou -NH₂.

3. Le dérivé peptidique représenté par la Formule (I) selon l'une des revendications 1 à 2, ou un stéréoisomère de celui-ci, ou un mélange de stéréoisomères de celui-ci, **caractérisé en ce qu'**il est sélectionné parmi les dérivés peptidiques suivants (1)-(4) :
(1) CA-Gly-Pro-Gln-Gly-Pro-Gln-OH ;
(2) CA-Gly-Pro-Gln-Gly-Pro-Gln-NH₂ ;
(3) GA-Gly-Pro-Gln-Gly-Pro-Gln-OH ;
(4) GA-Gly-Pro-Gln-Gly-Pro-Gln-NH_{2.}

4. Une composition cosmétique ou pharmaceutique, **caractérisée en ce qu'**elle inclut une quantité effective du dérivé peptidique représenté par la Formule (I) selon l'une des revendications 1 à 3, ou un stéréoisomère de celui-ci, ou un mélange de stéréoisomères de celui-ci, et au moins un excipient et optionnellement un adjuvant cosmétiquement ou pharmaceutiquement acceptable ;
optionnellement, l'adjuvant est sélectionné parmi un stimulateur de synthèse de collagène, un agent qui module la synthèse PGC-1α, un agent qui module l'activité de PPARγ, un agent qui augmente ou diminue la teneur en triglycérides dans les adipocytes, un agent qui stimule ou retarde la différenciation des adipocytes, un agent lipolytique ou un agent stimulant la lipolyse, un agent dégageant les graisses, un agent adipogénique, un inhibiteur contre l'agrégation des récepteurs de l'acétylcholine, un agent qui inhibe la contraction musculaire, un agent anticholinergique, un inhibiteur de l'élastase, un inhibiteur des métalloprotéinases matricielles, un stimulateur ou inhibiteur de la synthèse de la mélanine, un agent de blanchiment ou décolorant, un promoteur de pigmentation, un agent autobronzant, un agent anti-âge, un inhibiteur de NO-synthase, un inhibiteur de 5α-réductase, un inhibiteur contre l'hydroxylase lysyl et/ou l'hydroxylase prolyl, un antioxydant, un éliminateur de radicaux libres et/ou un agent contre la pollution atmosphérique, un éliminateur d'espèces carbonylées réactives, un agent anti-glycation, un antihistaminique, un agent antiviral, un agent antiparasitaire, un émulsifiant, un émollient, un solvant organique, un propulseur liquide, un conditionneur de peau, un humectant, une substance retenant l'humidité, un acide alpha-hydroxylé, un acide bêta-hydroxylé, un hydratant, une hydrolase épidermique, une vitamine, un acide aminé, une protéine, un pigment ou colorant, une teinture, un biopolymère, un polymère gélifiant, un épaississant, un tensioactif, un adoucissant, un adhésif, un conservateur, un agent anti-rides, un agent capable de réduire ou traiter les poches sous les yeux, un exfoliant, un agent éliminant les cuticules, un agent kératolytique, un antimicrobien, un antifongique, un fongistat, un bactéricide, un bactériostat, un agent qui stimule la synthèse de macromolécules dermiques ou épidermiques et/ou inhibe ou empêche leur dégradation, un agent qui stimule la synthèse de l'élastine, un agent qui stimule la synthèse de décorine, un agent qui stimule la synthèse de la laminine, un agent qui stimule la synthèse de défensine, un agent qui stimule la synthèse des protéines chaperons, un agent qui stimule la synthèse du cAMP, une protéine de choc thermique, un agent qui stimule la synthèse de HSP70, un agent qui stimule la synthèse d'une protéine de choc thermique, un agent qui stimule la synthèse de l'acide hyaluronique, un agent qui stimule la synthèse de la fibronectine, un agent qui stimule la synthèse de la désacétylase, un agent qui stimule la synthèse d'un lipide et d'un composant du stratum corneum, la céramide, un acide gras, un agent qui inhibe la dégradation du collagène, un agent qui inhibe la dégradation de l'élastine, un agent qui inhibe la sérine protéase, un agent qui stimule la prolifération des fibroblastes, un agent qui stimule la prolifération des kératinocytes, un agent qui stimule la prolifération des adipocytes, un agent qui stimule la prolifération des mélanocytes, un agent qui stimule la différenciation des kératinocytes, un agent qui inhibe l'acétylcholinestérase, un relaxant cutané, un agent qui stimule la synthèse des glycosaminoglycanes, un agent anti-hyperkératose, un agent comédolytique, un agent anti-psoriasique, un agent anti-dermatite, un agent anti-eczéma, un agent de réparation de l'ADN, un agent de protection de l'ADN, un stabilisateur, un agent antipruritique, un agent pour le traitement et/ou le soin de la peau sensible, un agent durcissant, un agent resserrant, un agent restructurant, un agent anti-étirement, un adhésif, un agent qui module la production de sébum, un antisudorifique, un agent qui stimule la guérison, un agent qui aide à la guérison, un agent qui stimule la ré-épithélialisation, un agent qui aide à la ré-épithélialisation, un facteur de croissance de cytokines, un sédatif, un agent anti-inflammatoire, un agent anesthésique, un agent agissant sur la circulation capillaire et/ou la microcirculation, un agent qui stimule l'angiogenèse, un agent qui inhibe la perméabilité vasculaire, un agent veinotonique, un agent agissant sur le métabolisme cellulaire, un agent améliorant la connexion derme-épiderme, un agent qui induit la pousse des poils, un agent inhibiteur ou retardant la croissance des cheveux, un parfum, un agent chélant, un extrait végétal, une huile essentielle, un extrait marin, un agent dérivé d'un processus de fermentation biologique, un sel inorganique, un extrait cellulaire, un écran solaire, et un photo-protecteur organique ou inorganique efficace contre les rayons UV A et/ou B, ou leurs mélanges.

5. La composition cosmétique ou pharmaceutique selon la revendication 4, **caractérisée en ce qu'**une préparation de composition cosmétique ou pharmaceutique est choisie parmi une crème, une huile, un lait, un baume, une mousse, une lotion, un gel, un liniment, un sérum, un savon, un shampoing, un après-shampoing, un sérum, un onguent, une mousse coiffante, une pommade, une poudre, une barre, un crayon, un spray, un aérosol, une capsule, un comprimé, un granulé, un chewing-gum, une solution, une suspension, une émulsion, un sirop, un élixir, un film de polysaccharides, une gelée ou une gélatine ;
optionnellement, la capsule comprend une capsule molle, une capsule dure, optionnellement une capsule de gélatine ;
optionnellement, le comprimé inclut un comprimé enrobé de sucre.

6. Un système d'administration cosmétiquement ou pharmaceutiquement acceptable ou système de libération prolongée, **caractérisé en ce qu'**il comprend une quantité effective du dérivé peptidique représenté par la Formule (I) selon l'une des revendications 1 à 3, ou un stéréoisomère de celui-ci, ou un mélange de stéréoisomères de celui-ci, ou la composition cosmétique ou pharmaceutique selon la revendication 4 ou 5 ;
Le système d'administration cosmétiquement ou pharmaceutiquement acceptable ou le système de libération prolongée est choisi parmi un liposome, un oléosome, une vésicule liposome tensioactif non ionique, un éthosome, une millicapsule, une microcapsule, une nanocapsule, un porteur lipidique nanostructuré, une éponge, une cyclodextrine, une vésicule lipoïde, une micelle, une millisphère, une microsphère, une nanosphère, une liposphère, une microémulsion, une nanoémulsion, une milliparticule, une microparticule ou une nanoparticule ; ou optionnellement un liposome ou une microémulsion ; ou optionnellement une microémulsion eau dans l'huile ayant une structure interne de micelle inverse.

7. Utilisation du dérivé peptidique représenté par la Formule (I) selon l'une des revendications 1 à 3, ou d'un stéréoisomère de celui-ci, ou d'un mélange de stéréoisomères de celui-ci, ou de la composition cosmétique ou pharmaceutique selon la revendication 4 ou 5, ou du système d'administration cosmétiquement ou pharmaceutiquement acceptable ou du système de libération prolongée selon la revendication 6 dans la préparation d'une composition cosmétique ou d'une composition pharmaceutique pour traiter, prévenir et/ou réparer les signes de vieillissement et/ou de photovieillissement de la peau.

8. Utilisation du dérivé peptidique représenté par la Formule (I) selon l'une des revendications 1 à 3, ou d'un stéréoisomère de celui-ci, ou d'un mélange de stéréoisomères de celui-ci, ou de la composition cosmétique ou pharmaceutique selon la revendication 4 ou 5, ou du système d'administration cosmétiquement ou pharmaceutiquement acceptable ou du système de libération prolongée selon la revendication 6 dans la préparation d'une composition cosmétique ou pharmaceutique pour l'élimination non thérapeutique des radicaux libres ou l'antioxydation.
